# EUROPEAN PATENT APPLICATION

(11) **EP 1 944 027 A1**
(43) Date of publication of application: **16.07.2008**
(21) Application number: 06798298.3
(22) Date of filing: 25.09.2006
(51) Int. Cl.: A61K 31/407, A61P 7/02, A61P 13/12, A61P 43/00, C07D 491/052, C12P 17/18, C12R 1/645

(54) **PHARMACEUTICAL COMPOSITION FOR TREATMENT OR PREVENTION OF NEPHRITIS AND METHOD FOR PRODUCING SAME**

(30) Priority: 06.10.2005 JP 2005293911
(71) Applicant: National University Corporation Tokyo University of Agriculture and Technology, Fuchu-shi, Tokyo 183-8538 (JP)
(72) Inventor: HASUMI, Keiji, Fuchu-shi, Tokyo 183-8538 (JP); YAGASAKI, Kazumi, Fuchu-shi, Tokyo 183-8538 (JP)
(74) Representative: Goddar, Heinz J.
(86) International application number: PCT/JP2006/318972
(87) International publication number: WO 2007/040082

(57) **Abstract**

A pharmaceutical composition for treatment of nephritis of the present invention comprises a triprenylphenol compound as an active substance, preferably the triprenylphenol compound represented by the following general formula (1) as an active substance, and further preferably orniplabin represented as n=3 in the following general formula (1) as an active substance. 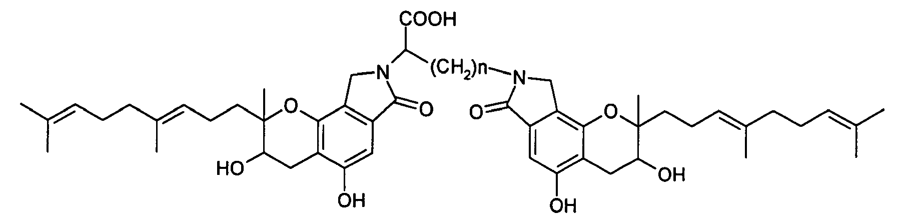 general formula (1) (in formula (1) n is one of integer selected from 1 to 10).

Further a manufacturing method of a pharmaceutical composition for treatment or prevention of nephritis of the present invention comprises using a triprenylphenol compound as an active substance.

## Description

### Technical Field

The present invention relates to a pharmaceutical composition for treatment or prevention of nephritis and a manufacturing method thereof.

### Background Art

Renal disorders can be mainly classified into two types, i.e. kidney inflammation (nephritis) and kidney failure. Nephritis is an inflammation of kidney. Kidney inflammation includes such interstitial nephritis and inflammation of renal pelvis, but in general, it is glomerulonephritis. Nephritis may cause a kidney failure in some cases. Kidney failure is a general disorder which results lowering kidney functions, and is mainly classified into acute kidney failure and chronic kidney failure. Most glomerulonephritis is due to antigen-antibody reaction, which may cause a pathologic change including such as deposition of fibrin in a glomerulus. In addition, according to progression of disorder of the kidney, especially nephritis, a deposition of extracellular stromal element is observed and an irreversible histological change occurs, and then kidney functions decline.

At present, an anti-inflammatory agent is administered to the treatment of nephritis. Such anti-inflammatory drugs are non-steroidal anti-inflammatory drugs (NSAIDs) such as aspirin and ibuprofen. In addition, kidney disorders are further categorized according to their cause, but a deposition of immune complex due to abnormal immunological reaction and an activation of complement and cellular immunity along with its deposition are commonly observed. From these standpoints, macrolide compounds to treat nephritis based on the TGF-β production-inhibitory activity and immunosuppressants have been developed (refer to Patent Reference 1 and Patent Reference 2). Further, nephritis is exacerbated by hemodynamic change-related stimulation by hyperglycemia and advanced glycation endproducts and subsequent growth response of endothelial and mesangial cells to the stimulus (proliferation, and production and release of cytokines, chemokines and/or growth factors). Furthermore, if it is progressed to glomerular sclerosis, it is known that an increase of production and deposition of extracellular matrix; suppressed production of MMPs and plasmin; and an increased production of PAI-1, and tissue inhibitor of metalloprotease (TIMP) occur (refer to non-patent reference 1).

On the other hand, a variety of triprenylphenol derivatives obtained from filamentous fungus have been disclosed to interactively enhance the fibrin binding of plasminogen and its activation to a plasmin. (Patent References 3 through 5) Accordingly, the triprenylphenol derivatives may be employed as a drug for treatment or prevention of thrombotic diseases enhancing an induction of clot lysis either with or without a known thrombolytic drug.
[ Patent Reference 1] Japanese Patent Application Laid-open (JP-A) No. 2003-137791
[Patent Reference 2] JP-A No. H06-316588
[Patent Reference 3] JP-A No.JP2002-65288
[Patent Reference 4] JP-A No. JP2003-88397
[Patent Reference 5] JP-A No. JP2004-224737
[Non-Patent Reference 1] "Prospective Overview for Progression Mechanisms and Treatments of Damaged Glomerulus" Igaku-no-ayumi, 2004, vol. 209, pp33 - 38

### Disclosure of the Invention

### Problems to be Solved by the Invention

It is known that NSAIDs used to treat nephritis has a cyclooxygenase inhibitory activity which may cause adverse effect. The adverse effects due to such NSAIDs are stomach pain and headache due to an inhibition of prostaglandin synthesis. Further according to the strength of activity of NSAIDs to kidney they may cause a kidney failure. In contrast, no relationship between a triprenylphenol compound and nephritis is known.
Accordingly, an object of the present invention is to provide a pharmaceutical composition for treatment or prevention of nephritis which has excellent effects on nephritis treatment or prevention and has fewer adverse effects. Another object of the present invention is to provide a manufacturing method thereof.

A pharmaceutical composition for treatment or prevention of nephritis of the invention comprises a triprenylphenol compound as an active substance.
A manufacturing method of a pharmaceutical composition for treatment or prevention of nephritis of the invention comprises using a triprenylphenol compound as an active substance.
In the pharmaceutical composition and the manufacturing method thereof, the triprenylphenol compound is preferably at least one selected from the group consisting of compounds represented by the following general formula (1), general formula (2) and formula (3).

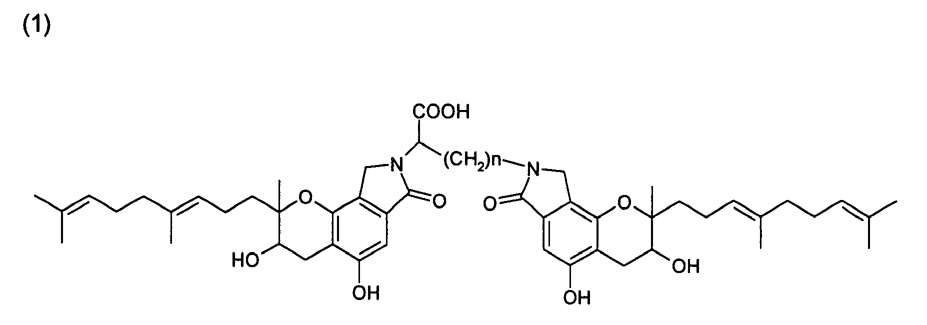

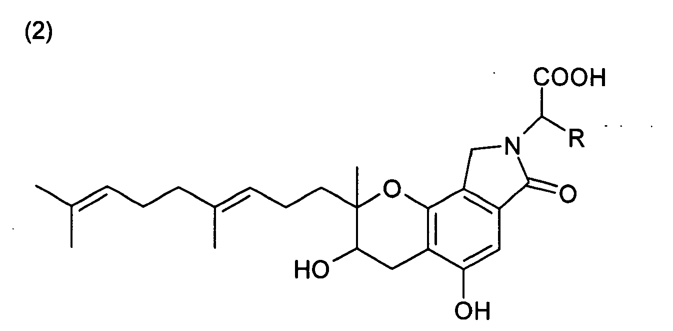

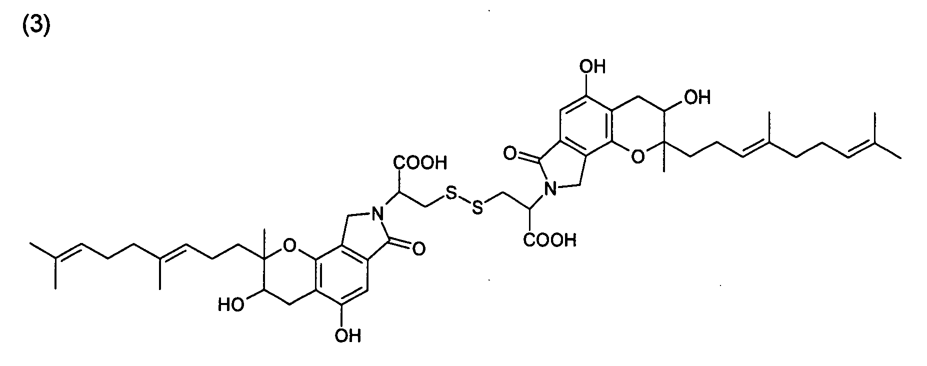

n in general formula (1) is an integer between 1 and 10 and R in general formula (2) is one of groups represented in the followings.

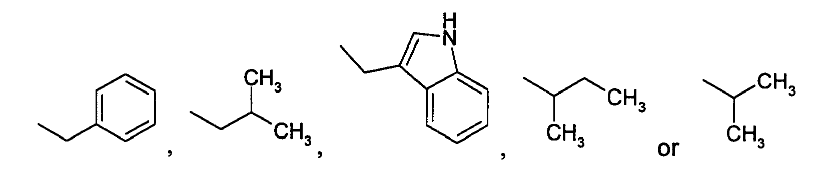

Further it is preferable that the above triprenylphenol compound is obtained from a filamentous fungus.

The pharmaceutical composition for treatment or prevention of nephritis of the invention comprises a triprenylphenol compound, preferably at least one triprenylphenol compound selected from the group consisting of the compounds represented by the above general formula (1), general formula (2) and formula (3) as an active substance.

### Effects of the Invention

According to the present invention, a pharmaceutical composition for treatment or prevention of nephritis which has excellent effects on nephritis treatment or prevention and has fewer adverse effects can be provided. Further according to the present invention a manufacturing method thereof can be provided.

### Brief Description of the Inventions

Fig.1 is a graph representing the effect of orniplabin on proteins in urine according to an example of the present invention.
Fig.2 is a graph representing the effect of orniplabin on lipid peroxides in blood according to an example of the present invention.
Fig.3 is a graph representing the effect of orniplabin on the weight of lever according to an example of the present invention.
Fig.4 is a graph representing the effect of orniplabin on the weigh of kidney according to an example of the present invention.
Fig.5 is a graph representing the effect of orniplabin on the concentration of plasma constituents according to an example of the present invention.
Fig.6 is a graph representing the effect of orniplabin on the concentration of other plasma constituents according to an example of the present invention.
Fig.7 is a graph representing the effect of orniplabin on the concentration of other plasma constituents according to an example of the present invention.
Fig.8 is a graph representing the effect of orniplabin on the concentration of other plasma constituents according to an example of the present invention.
Fig. 9A is a PAS stained image of a normal portion of glomerulus of the normal group according to an example of the present invention.
Fig. 9B is a PAS stained image of the pathologically changed portion due to nephritis of glomerulus of the positive control group according to an example of the present invention.
Fig. 9C is a PAS stained image of the pathologically changed portion due to nephritis of glomerulus of the orniplabin administered group according to an example of the present invention.
Fig. 10A is a PAS stained image of the pathologically changed portion due to nephritis of renal tubular of the positive control group according to an example of the present invention.
Fig. 10B is an expanded figure of Fig. 10A.

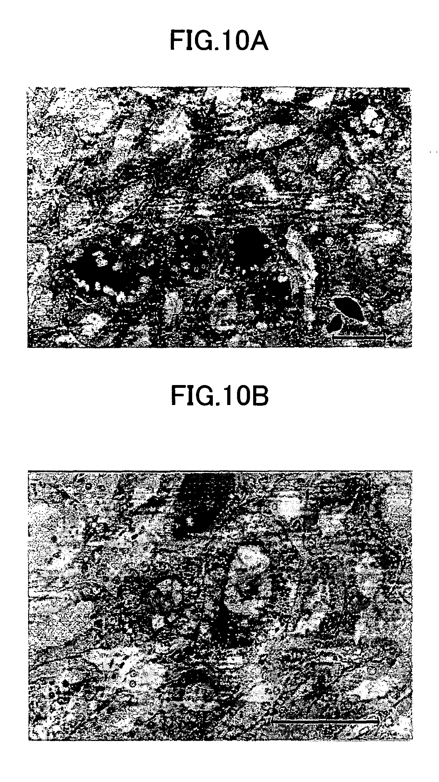

Fig. 10A is a PAS stained image of the pathologically changed portion due to nephritis of renal tubular of the ornilabin administered group according to an example of the present invention.

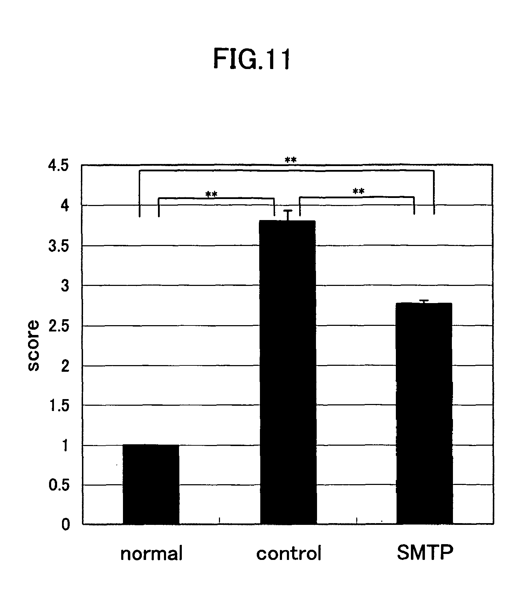

Fig. 11 is a graph representing the effect of the orniplabin administration based on the nephritis score according to an example of the present invention.

### Best Mode for Carrying Out the Invention

A pharmaceutical composition of the present invention for treatment or prevention of nephritis comprises a triprenylphenol compound, preferably at least one of the selected compounds represented by the above general formula (1), general formula (2) or formula (3) as an active substance.

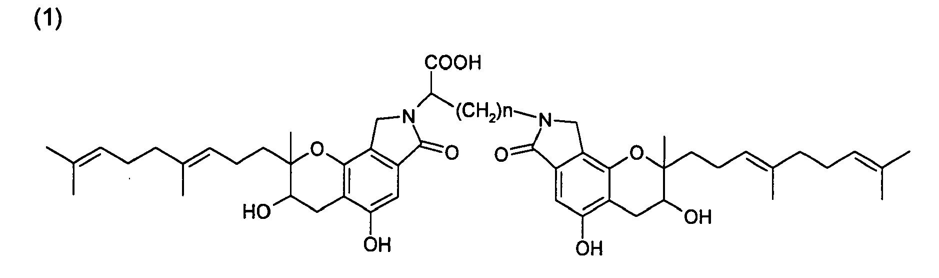

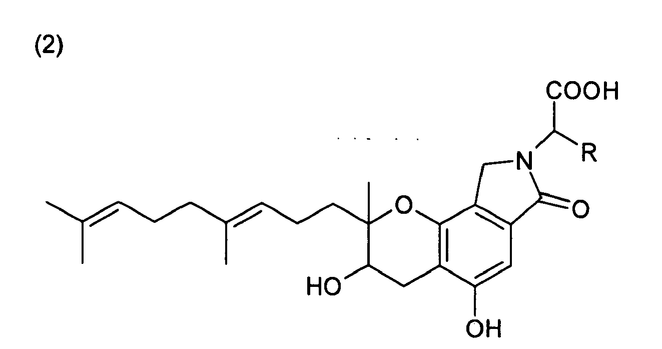

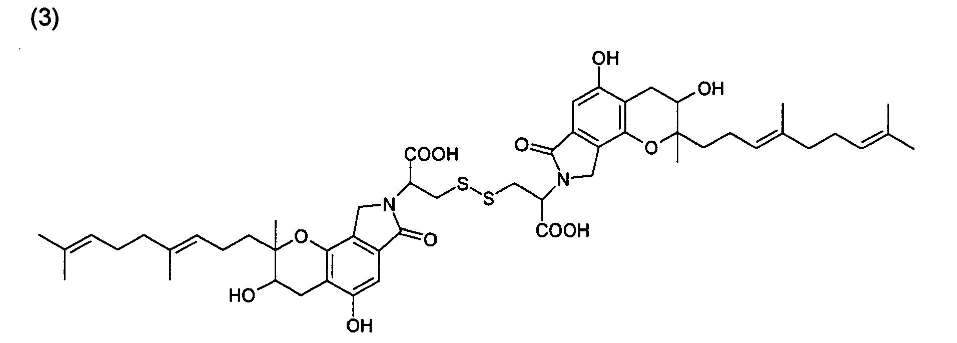

n in general formula (1) is an integer between 1 and 10 and R in general formula (2) is one of groups represented in the following.

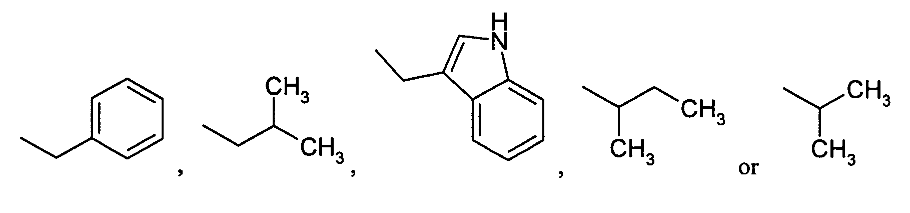

From a treatment effect of nephritis and an enhancement effect of plasminogen activation standpoints, the above triprenylphenol compound is preferably at least selected one of the compounds represented in general formula (1), of which n is an integer between 2 and 7, and the following compounds (2) or (3).

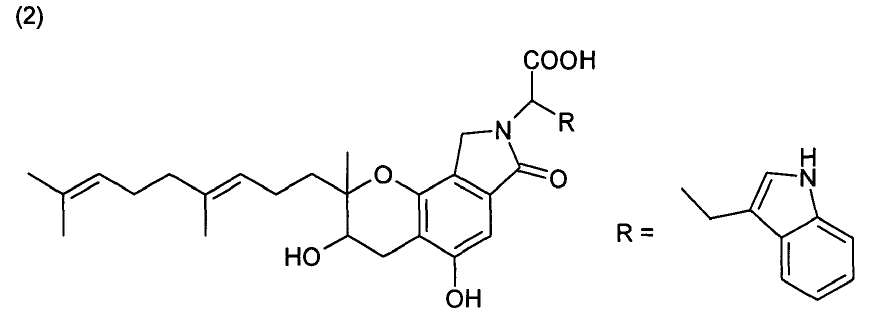

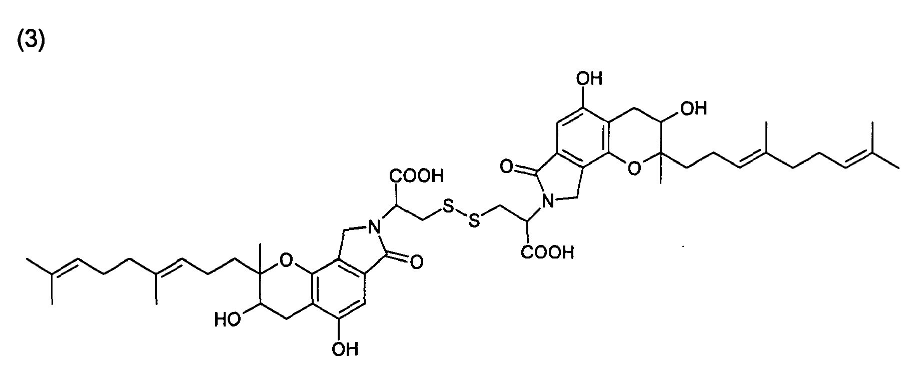

Especially, triprenylphenol compounds having n between 2 and 4 in general formula (1) is more preferable because of stronger treatment effect of nephritis and enhancement effect of plasminogen activation. Among them, orinplabin shown below, of which n is 3, is further especially preferable because of higher inhibitory effect on nephritis.

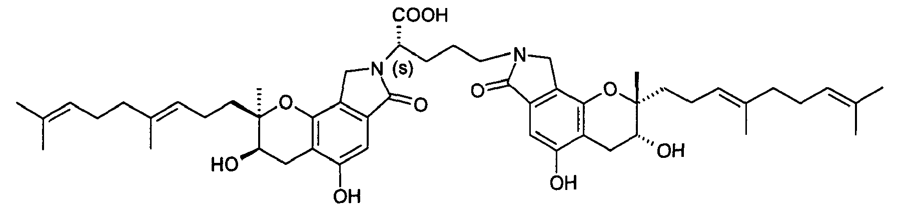

Most glomerulonephritis is due to antigen-antibody reaction, which may cause a pathologic change including such as deposition of fibrin in a glomerulus. In addition, according to progression of nephritis, a deposition of extracellular matrix composition is observed and an irreversible histological change occurs, and then kidney functions decline.
A triprenylphenol compound of the present invention has fibrinolytic reaction enhancement activity and is known as a compound which gives less adverse effect, and in addition, the inventors discovered that the triprenylphenol compound of the present invention may enhance a lysis process of anti-kidney basement membrane antibody itself or an immune complex of the host corresponding to anti-kidney basement membrane antibody, or a proteolysis of the local tissues because of the enhancement effect of fibrinolytic reaction.
Accordingly, a pharmaceutical composition comprising a triprenylphenol compound represented by the above general formula as an active substance may treat or prevent nephritis.
A pharmaceutical composition of the present invention may comprise one of the triprenylphenol compounds represented in the above formulae (1) through (3) or may comprise a combination of more than two compounds. Further, the composition may comprise a single compound or plural compounds.

A triprenylphenol compound of the present invention may be synthesized using an organic chemistry process, e.g. chemically derived from a compound having a prenylphenol unit.
Further, it is preferable that a triprenylphenol compound of the present invention is a metabolite obtained from culture of a filamentous fungus. A detail method to obtain a triprenylphenol compound of the present invention is disclosed in JP-A Nos. 2002-65288 and 2004-224737..
Specifically, a preferable manufacturing method comprises a step of fermenting a filamentous fungus in liquid medium, a step of extracting a triprenylphenol compound of the present invention from the culture, and a step of using the extracted triprenylphenol compound as an active substance of a pharmaceutical composition.
A method to obtain the triprenylphenol compound of the present invention may be an extraction method applied, as usual, to such culture of filamentous fungus. The extraction method can be applied is e.g. a liquid-liquid partition method using ethyl acetate and 2-butanone, precipitate formation by adjusting pH of the culture filtrate to approximately 3 with such as phosphoric acid and/or hydrogen chloride, and adsorption method in which the culture filtrate contacts to a hydrophobic adsorption resin.

The genus of a filamentous fungus employed to produce a triprenylphenol compound of the present invention is Stachybotrys genus and a filamentous fungus of Stachybotrys genus can be preferably selected. Further preferably the filamentous fungus is *Stachybotrys microspora,* IFO030018 but the present invention is not limited to this strain.
A base culture medium is preferably the medium A but not limited to. Medium A is composed of 20g of glucose, 5g of peptone, 3g of yeast extract, 3g of dipotassium phosphate, 1g of magnesium sulfate heptahydrate and 1 L of water, and its pH is adjusted to 5.5 with hydrogen chloride or sodium hydroxide.

As to the medium composition, appropriate amino acid, amino alcohol or amine can be preferably added to the medium according to the structure of the targeted triprenylphenol compound. For example, L-ornithine is added to provide orniplabin and a compound having the below general formula (4), wherein R is the same as in general formula (1), is added to the medium to provide a compound having general formula (1), or a compound having below general formula (5) is added to the medium to provide the compound having general formula (2). Further L-cystine or L-cysteine, especially L-cystine, is added to the medium to provide compound (3) having formula (3). Accordingly, an appropriate compound can be added to selectively provide the triprenylphenol compound of the present invention.

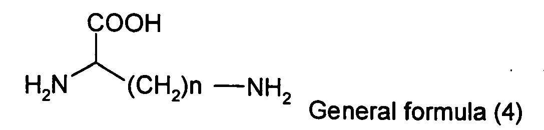

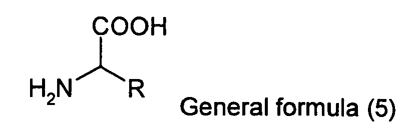

A concentration of amino acid or amino alcohol added is preferably in the range of 0.5 mg/ml and 2 mg/ml, but the concentration of the present invention is not limited to. Addition of amino acid or amino alcohol is preferably carried out between at the time immediately after begging of culture and by three days after begging of culture. Optimal culture temperature is 25°C but not limited to this temperature. A culture period is three to six days after addition of amino acid or amino alcohol and accordingly a satisfactory production amount of triprenylphenol compound can be obtained. An appropriate aeration and stirring condition are the conditions that can be provided by 180 rpm rotation culture if 500 mL of Erlenmeyer flask having an appropriate aeration stopper is used with 100 mL of medium A. If a jar-fermenter is used, the same aeration and stirring condition employed for the above cultivation conditions with an aeration stopper is preferred.

A triprenylphenol compound of the present invention can be used as a free form, a pharmacologically acceptable salt, an ester, or a solvated form thereof. In such case, a manufacturing method of the pharmaceutical composition of the present invention may comprise a manufacturing step of manufacturing respective forms.
Inorganic acid such as hydrochloric acid, hydrobromic acid, sulfuric acid, nitric acid, and phosphoric acid; or organic acid such as citric acid, formic acid, fumaric acid, malic acid, acetic acid, succinic acid, tartaric acid, methanesufonic acid, and p-toluenesulfonic acid are adequate to form a pharmacologically acceptable salt of orniplabin. Further, for example, a compound including alkali metal or alkali earth metal such as sodium, potassium, calcium, and magnesium; and one selected compound from a group composed of basic amines and basic amino acids are also adequate to form such pharmacologically acceptable salts.
If a salt of triprenylphenol compound is formed according to the present invention, for example, the above inorganic salt or respective compounds and the triprenylphenol compound can be mixed in the ratio of 1:10 to 1:1 by mass, but not limited to.
Further, one selected compound from alcohols and carboxylic acids having 1 to 10 carbon(s), preferably a compound such as methyl alcohol, ethyl alcohol, acetic acid and propionic acid is adequate to form a pharmacologically acceptable ester. If an ester of triprenylphenol compound is formed according to the present invention, for example, the above alcohol and carboxylic acid compound and the triprenylphenol compound can be mixed in the ratio of 1:1 to 10:1 by mass, but not limited to.
Further, such as water is adequate to form a pharmacologically acceptable solvated compound of the compound of the present invention. A solvated compound of a triprenylphenol compound according to the present invention, for example, the triprenylphenol compound can be dissolved in the concentration of 1 mg/mL to 100 mg/mL, but not limited to.

The pharmaceutical composition for treatment or prevention of the present invention can be administered orally or non-orally, e.g. an intraperitoneal administration to be able to directly deliver the pharmaceutical composition of the present invention to the kidney is also an effective route. Further, a formulation of the pharmaceutical composition for treatment or prevention of the present invention can be changed according to the selected administration route. For example, tablet, capsule, powder, fine particle, granule, liquid, and syrup can be an appropriate oral administration formula; and injection, drip infusion, suppository, respiratory tonic, and patch can be an appropriate non-oral administration formula.
A dose of the triprenylphenol compound is not specifically limited and it can be decided from case by case according to an administration formula, age, body weight and symptoms. For example, if an intravenous injection is selected, the effective dose for an adult per day is preferably between 1 mg/Kg body weight and 50 mg/Kg body weight, and if an intraperitoneal administration is selected, the effective dose is between 1 mg/Kg body weight and 50 mg/Kg body weight, preferably between 5 mg/Kg body weight and 15 mg/Kg body weight, and further preferably 10 mg/Kg body weight. If an oral administration is selected; the effective dose for an adult per day is preferably between 2 mg/Kg body weight and 200 mg/Kg body weight. A dosing period can be discretionally decided according to age and symptoms. Content of the triprenylphenol compound in the pharmaceutical composition for treatment or prevention of the present invention can be changed from case by case according to the formula of the composition and the effective dose after administered according to the description of the present invention.

A pharmaceutical composition for treatment or prevention of the present invention may comprise a pharmacologically acceptable carrier according to the formula thereof. Such carrier can be a variety of organic or inorganic carriers which are commonly employed for such use. Further it can be such as pharmaceutical vehicle, lubricant, binding agent, and disintegrating agent for a solid medicine; such as solvent, solubilizing agent, suspending agent, tonicity agent, buffer agent, and soothing agent for a liquid medicine. Further, according to necessity, an additive such as antiseptic agent, antioxidant, coloring agent, sweetener, adsorbing agent and humecatant which are commonly used can be contained.

A pharmaceutical composition for nephritis treatment or prevention of the present invention can be used to treat not only a selected nephritis from interstitial nephritis, pyelonephritis, and glomerular type nephritis but also to treat functional damages of kidney due to such nephritis. Such functional damages of kidney due to such nephritis, for example, include acute kidney failure and chronic kidney failure.
Further a pharmaceutical composition for nephritis treatment of the present invention is a pharmaceutical composition to suppress or relieve the progress of symptoms when a symptom is found due to nephritis. On the other hand, a pharmaceutical composition for nephritis prevention of the present invention is a pharmaceutical composition to suppress an occurrence of symptom, which is predicted due to nephritis, with pre-administration. However, it should be used based on multiple aspects including the timing of use and/or the symptom on use and it should not be restrictively applied.

### Example

According to one example of the present invention orniplabin as an example is described but not limited to. The % represents weight (mass) percent unless it is remarked in particular.

### Example 1

### (1) Synthesis of orniplabin

Orniplabin is obtained according to JP-A No. 2004-224737. Specifically, a loopful of *S. microspora* IFO30018 on the slant culture was inoculated into the 100 mL of medium containing 3% of glucose, 1% of soybean powder, 0.3% of peptone, 0.3% of meat extract, 0.3% of yeast extract, 0.05% of KH₂PO₄, 0.05% of MgSO₄/7H₂O, and 0.01% of CB 442 (antifoaming agent, Nihon Yushi, Japan) in 500 mL Erlenmeyer flask. The flask was incubated for 3 days at 25°C on the rotary shaker rotating at 180 rpm. The culture obtained from this incubation was used as the seed culture. One mL of this seed culture was inoculated into the 100 mL of medium containing 2% of glucose, 0.5% of peptone, 0.3% of yeast extract, 0.3% of KH₂PO₄ 0.1% of MgSO₄/7H₂O, 100 mg of ornithine as an amino acid and 0.01% of CB 442 (pH 5.5) in 500 mL Erlenmeyer flask. As described above, the flask was incubated for 4 to 6 days.
The supernatant of the culture was extracted with 2-butanone, and the extract was dried over anhydrous sodium sulfate and evaporated. The oily residue was dissolved in methanol to provide approximately 100 mg/mL solution, and the solution was passed through RP-18 solid phase column (LiChrolut®) and subjected to preparative HPLC with Intersil PREP-ODS column (30 x 250mm; GL Science (Tokyo, Japan). The column was developed with 50 mM ammonium acetate in 80% aqueous methanol and with a flow-rate of 25 mL/min at 40°C, and the fraction eluted between 34 and 39 minutes gave orniplabin of one example of the present invention.

### Example 2

### (1) Effects of orniplabin on urinary protein, level of lipid peroxide in blood, and organomegaly

Three-week-old male Wister rats (Charles River) were raised for 6 days with pellet diet (CE-2, Nihon Crea) and then raised with the 20% casein diet for 5 days to be subjected to the experiment. The animals were raised at approximately 22°C, with relative humidity 60±5° under lighting from 8:00 to 20:00. The formula of casein diet is shown in Table 1.

**Table 1**

| | |
|---|---|
| Casein (Oriental yeast) | 20% |
| Corn oils (Hayashi Chemical) | 5% |
| α cornstarch | 68.3% |
| Mineral mixture (Composition of AlN-76, Nihon Noyaku Industry) | 3.5% |
| Vitamin mixture (Composition of AlN-76, Nihon Noyaku Industry) | 1 % |
| Choline di-tartaric acid (Wako Junyaku Industry) | 0.2% |
| Cellulose powder (Oriental yeast) | 2% |
| Total | 100% |

0.6 mL of Anti-rat-glomerular basement membrance rabbit serum was administered into the caudal vein. Next day rabbit γ-globulin (Sigma), and 8 mg/0.2 mL Freund's complete adjuvant (Wako Junyaku) were subcutaneously injected into hinder leg's footpad to cause nephritis (nephritis model rat).
Since anti-serum was injected, the body weight and feed intake were measured every morning and urinary proteins of 24-hour urine was measured every other day, specifically urine was collected in a flask from 9AM of the day before to 9AM of the day and the measurement was carried out using commercial protein measurement kit (Bio-Rad, Protein Assay, Bio-Rad). (Refer to Biosci. Biotechnol. Biochem., 65, 1155-1162 (2001))

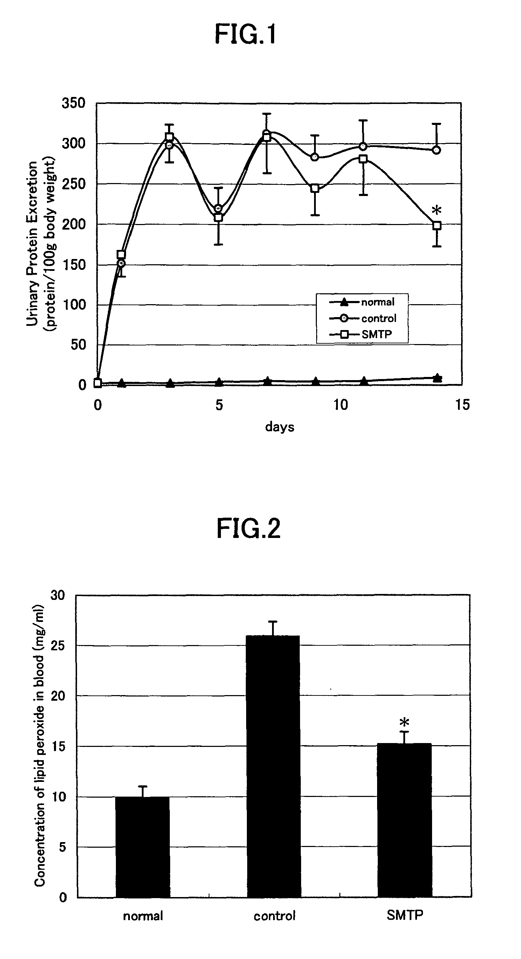

The urinary proteins were measured three days after the injection of anti-serum and the rats were grouped according to the measurement values. An orniplabin solution equivalent to 5 mg/Kg concentration was intrapenitoneally injected everyday to the orniplabin administration group since then. Nothing was injected to the control group. Rats of respective groups were dissected to remove blood, liver and kidneys 14 days after nephritis was induced, and weights of the removed materials were measured. Further lipid peroxides of a part of blood obtained were measured using a commercial measurement kit (Wako Junyaku). The normal group represents the untreated group, the control group represents - the nephritis-induced group and SMTP group represents the orniplabin-injected group. Student's t-test of significant difference between two groups was carried out as the level significance is 0.05. Tukey's multiple comparison method was used for the testing of significance between three groups. The data associated with the amount of urinary proteins and the weight of organs are treated with the scale per 100g of body weight as a benchmark..
The results are shown in Fig. 1 to Fig. 4. The asterisk, *, in Fig. 1 to Fig.4 indicates that it was significant as compared with the control (p<0.05).

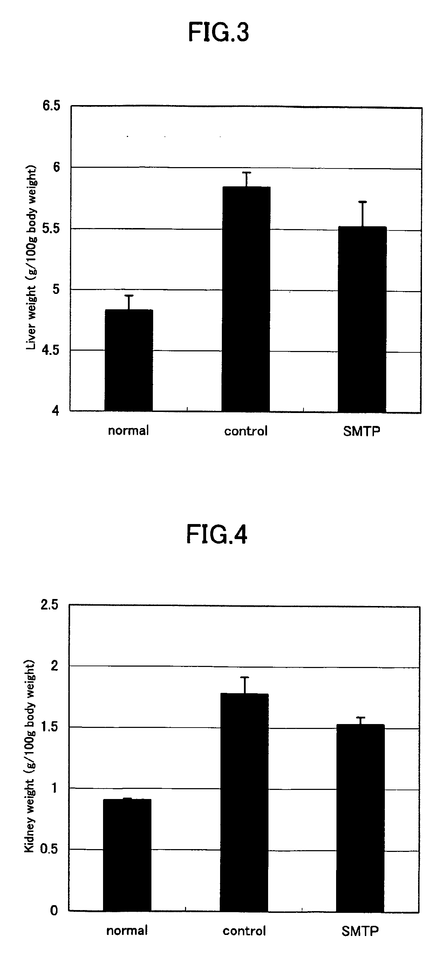

Referring to Fig. 1 to Fig. 4, the administration of orniplabin to the nephritis model rats decreased urinary proteins (Fig. 1), decreased lipid peroxides in serum (Fig. 2), and suppressed hypertrophy of liver (Fig. 3) and kidneys (Fig. 4) and the nephritis suppression effect of orniplabin were shown from these results. Especially the initial change of the amount of urinary proteins was small but the difference between the control group and the orniplabin-injected group became big. In contrast, there is no increase of the body weight and the effect on food intake. (No data is shown.) These results indicate that orniplabin enhanced the recovery from nephritis.

### (2) Effects of orniplabin administration on plasma components

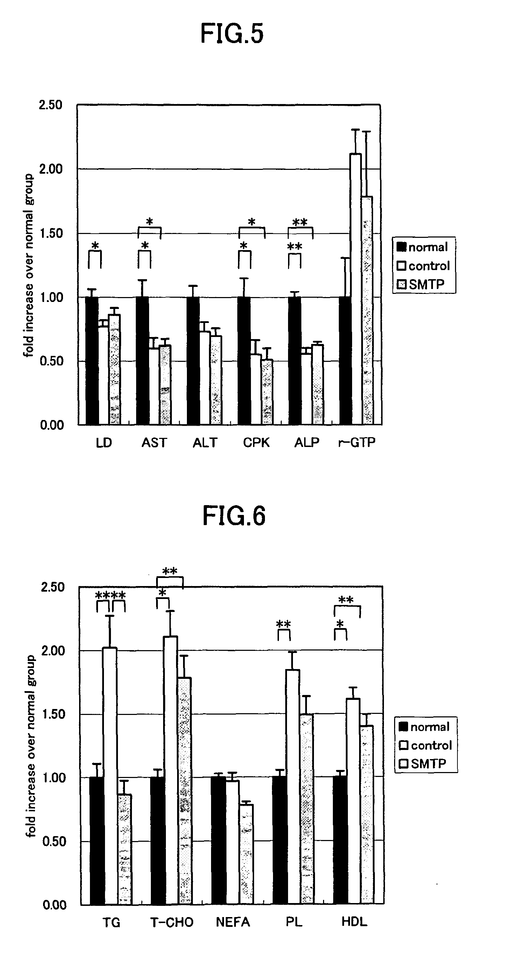

Further effects of orniplabin administration on plasma components were investigated by measuring a variety of concentrations of plasma component, (Normal group, n = 6; control group, n = 10; and orniplabin-injected group, n = 6) The following plasma components were measured; lactate dehydrogenase (LD; PureAuto S LD), asparagine amino transferase (AST AutoSera S AST), alanine aminotransferase (ALT: AutoSera S ALT), creatine phosphate kinase (CPK; PureAuto S CK), alkaline phosphatase (ALP; AutoSera S ALP), γ-glutamyl transpeptidase (γ-GTP; PureAuto S γ-GT), total bilirubin (BIL: AutoSera BIL-2), glucose (GLU; PureAuto S GLU-R), triglyceride (TG; AutoSera S TG-N), total cholesterol (T-CHO; Choletest CHO), phospholipid (PL; PureAuto S PL), total protein (TP; AutoSera TP), urea nitrogen (UN; PureAuto S UN), albumin (ALB; AutoSera ALB), calcium (CA; AutoSera CA), inorganic phosphoric acid (IP; Clini mate IP-2), (Daiichi Chemical), creatinine (CRE-S; AccursAuto CRE, Sinotest), acetoacetic acid (ACAC; Ketone Test A Sanwa liquid, Sanwa Science Laboratories), 3-hydroxy butyric acid (OHBA; Ketone Test B Sanwa liquidA Sanwa Science Laboratories), free fatty acids (NEFA; AutoSera R NEFA (Daiichi Chemical) and HDL cholesterol (HDL; Cholest-R N HDL (Daichi Chemical) were used to measure. Further Hitachi Amino acid automatic analyzer 7180 (Hitachi) was used.
The comparison results between the control group and the orniplabin-injected group, and the normal group of which average value is as 1 are shown in Fig. 5 to Fig. 8.
Further * and ** in Figs. indicate the level of significance between the indicated groups is less than 0.05 and 0.01 respectively.

The concentrations of LD, AST, ALT, OK, ALP and γ-GTP but γ-GTP in plasma of the nephritis-induced group (control group) were lower than those in plasma of normal animals (refer to Fig. 5). These differences in values between the animals (orniplabin-injected group) to which orniplabin (SMTP-7) was injected after induction of nephritis and the control group were not significantly different. In contrast, it was shown that the concentrations of TG, T-CHO, NEFA, PL and HDL but NEFA in plasma of the nephritis-induced group increased respectively. When the orniplabin was injected, TG significantly decreased, and T-CHO, PL and HDL were in decreasing trend. This indicated that the secretion of respective factors into the blood was in releasing trend to some extent by the administration of orniplabin (refer to Fig. 6). In particular, the triglyceride value was indicated to become normal level by the orniplabin administration. These results indicated that orniplabin might decrease the concentrations of lipids and cholesterol in plasma.

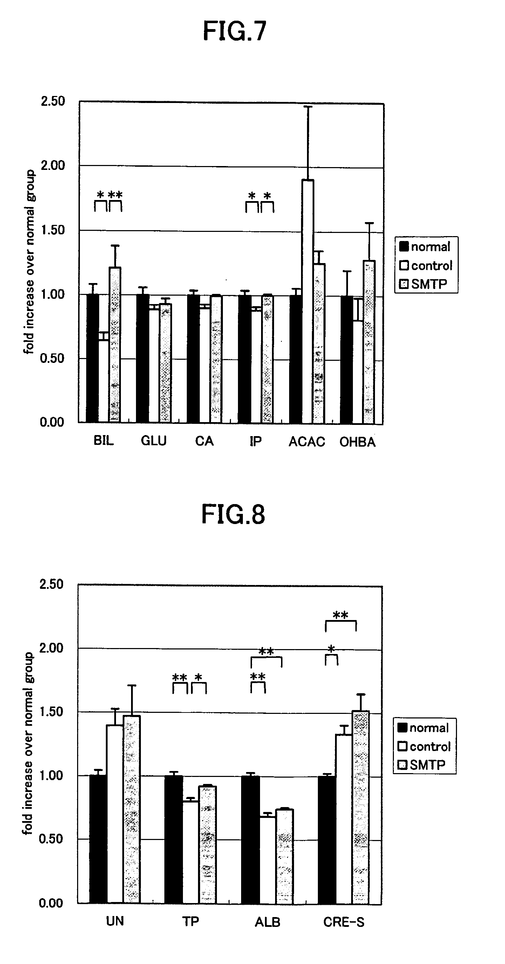

On the other hand, it was indicated that the concentration of BIL, GLU, CA, IP and ACAC and OHBA but ACAC (acetoacetic acid) in plasma were in decreasing trend respectively by the induction of nephritis but the decrease of these concentration in plasma was prevented or these concentrations were recovered by the orniplabin administration.
The concentration of ACAC in plasma of the orniplabin-injected group was lower than that of the control group and, in contrast, the concentration of OHBA in plasma of the orniplabin -injected group was higher than that of the control group. These two components are convertible each other in liver and secreted into plasma so that the higher acetoacetic acid was the lower hydroxybutyric acid every animal.
The concentrations of UN, ALB and CRE-S were almost not different between the control group and the orniplabin-injected group, but the concentration of TP (total proteins) were recovered to the normal level by the administration of orniplabin (refer to Fig. 8).
According to these results, orniplabin may suppress hyperlipidemia due to nephritis and treat the damages due to re-assorption decrease of inorganic substance such as calcium.

### (3) Observation of histologic section

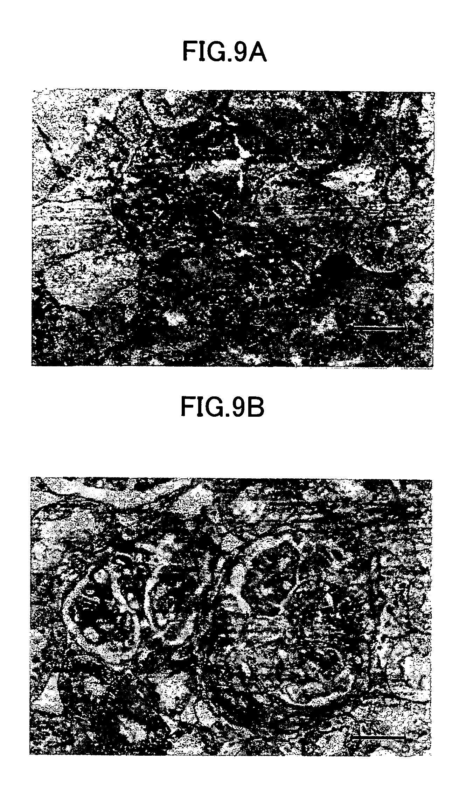

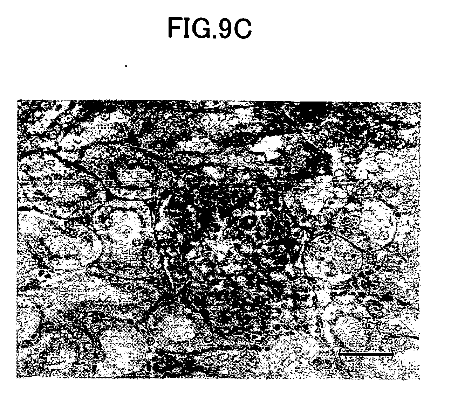

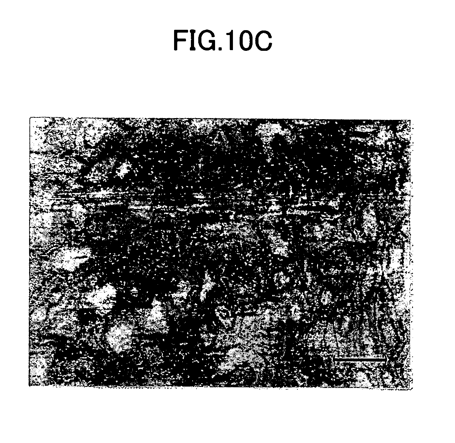

Section samples were prepared from three animals selected according to the variation of urinary protein in respective groups. Periodic acid Schiff (PAS) staining was conducted on the kidney histologic section.
A procedure for PAS staining was; slicing a sample, washing the sample with water after paraffin-removal, soaking the sample in a solution of 1% periodic acid for 10 minutes, and washing again the sample with water; soaking the histologic section prepared in Schiff reagent for 10 to 15 minutes, washing the sample in a staining bat three times for 3 minutes, total for 9 minutes, and washing the sample with water; and then staining the sample with hematoxylin for 1 minute, washing the sample with warm water for 10 minutes, dehydrating and penetrating the sample and then sealing the sample.
Observation of the histologic sample was conducted on renal glomerulus using a microscope with 200x magnifications. The results are shown in Fig. 9 and Fig. 10. The bar in Figs is corresponding to 50 µm.

Referring to Fig. 9, the control group (refer to Fig. 9B) showed obvious crescent formation and such typical symptoms due to anti-glomerulonephritis as mesangium proliferation, but, in contrast, the orniplabin-injected group (refer to Fig. 9C) showed a low level crescent formation and almost no mesangium proliferation although the similar symptoms were observed. Further referring to Fig. 10, some animals showed abnormal histologic hypertrophy of proximal convoluted tubule tissue (refer to Fig. 10A and 10B) but such hypertrophy was not observed in the orniplabin-injected group. These symptoms almost were not observed in the normal group (refer to Fig. 9A).

Fifty (50) glomeruli every three samples of each group were ranked according to damaged levels. The ranks were from normal (rank 1) to the most progressed damage (rank 5) and five grades according to damages were given to provide a score. The average of the scores was decided as the score of the sample (nephritis score). The results are shown in Fig. 11. Further, ** in Fig. 11 indicates that there was significant difference (p<0.001).
Referring to Fig. 11, the control group had 3.80±0.13 versus 2.77±0.04 for the orniplabin -injected group, in which the lowering score was statistically significant.
These results showed that orniplabin had a nephritis inhibitory effect.

According to examples of the present invention, an administration of orniplabin lowered an amount of urinary proteins, lipid peroxides in plasma and triglycerides without affecting to the increase of body weight and the food intake, and recovered the amounts of bilirubin, inorganic phosphoric acid, and total proteins to the normal level. The masses of kidneys and liver increased due to induction of nephritis but the administration of orniplabin showed a decreasing trend for these masses. An extra tubular proliferative nephritis characterized by crescent formation and mesangium proliferation which were observed in the control group was inhibited by the administration of orniplabin. Further according to the observation of damages in glomeruli the administration of orniplabin significantly lowered the nephritis scores.
Accordingly, a pharmaceutical composition of the present invention comprising a triprenylphenol compound as an active substance is remarkably effective on treatment or prevention of nephritis.

The entire disclosures of Japanese Patent Application No. 2005-293911 are herein fully incorporated. As specifically described in respective literatures, patent applications and technological specifications, all literatures, patent applications and technological specifications in the description of the present invention are incorporated in the description of the present invention.

## Claims

1. A pharmaceutical composition for treatment or prevention of nephritis, comprising a triprenylphenol compound as an active substance.

2. The pharmaceutical composition for treatment or prevention of nephritis according to claim 1, wherein:
the triprenylphenol compound is at least one selected from the group consisting of compounds represented by the following general formula (1), general formula (2) or formula (3). 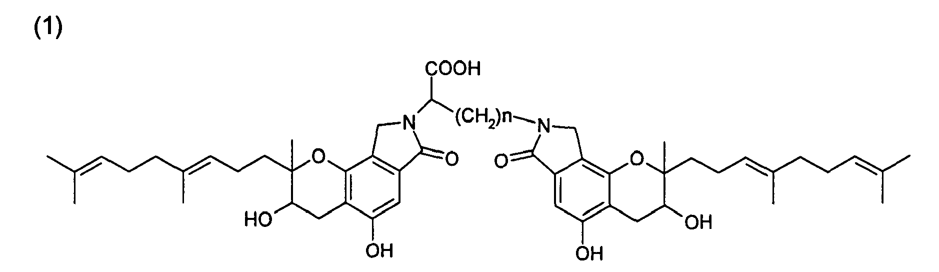 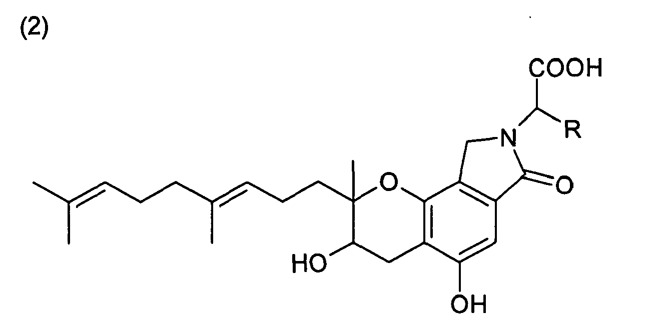 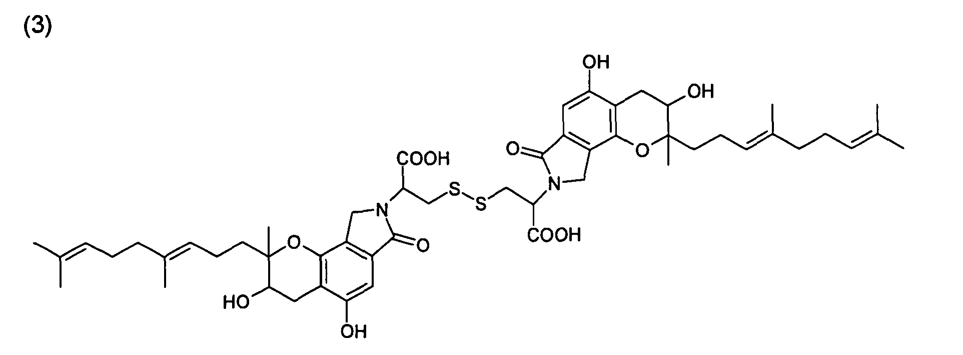 (In the above formulae, n is an integer of from 1 to 10 and R is one of following.)

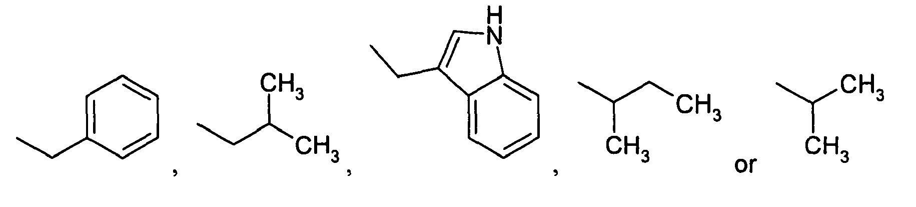

3. The pharmaceutical composition for treatment or prevention of nephritis according to claim 1: wherein
the triprenylphenol compound is represented by the general formula (1).

4. The pharmaceutical composition for treatment or prevention of nephritis according to claim 2 or claim 3, wherein
n in the general formula (1) is from 2 to 7.

5. The pharmaceutical composition for treatment or prevention of nephritis according to claim 1: wherein
the triprenylphenol compound is at least one of following compounds.

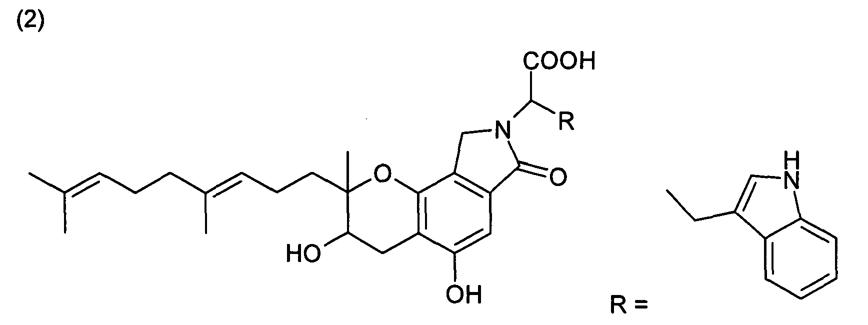

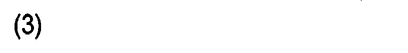

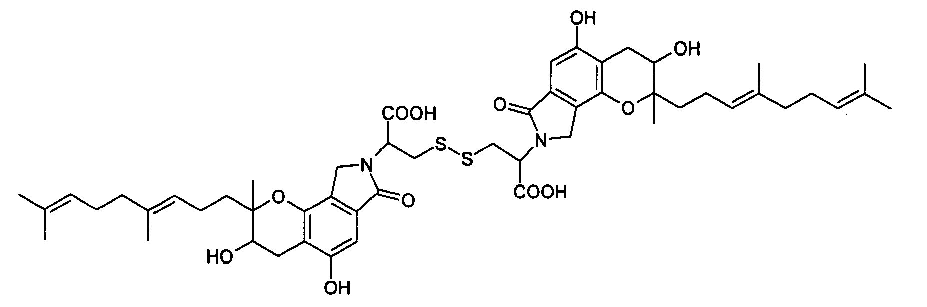

6. The pharmaceutical composition for treatment and/or prevention of nephritis according to claim 1, wherein
the triprenylphenol compound is the following compound.

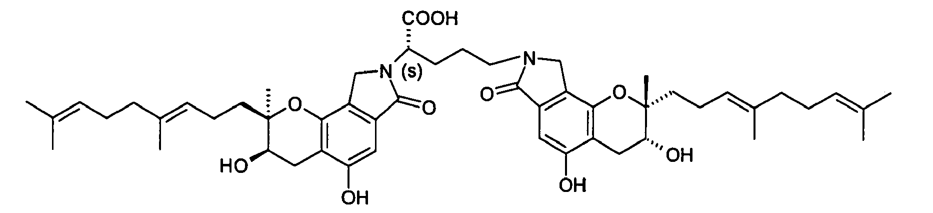

7. The pharmaceutical composition for treatment or prevention of nephritis according to claim 1, wherein
the triprenylphenol compound is a pharmacologically acceptable triprenylphenol compound obtained by using at least one compound selected from the group consisting of inorganic acids, organic acids, compounds including an alkali metal or an alkali earth metal, basic amines and basic amino acids.

8. The pharmaceutical composition for treatment or prevention of nephritis according to claim 1, wherein
the triprenylphenol compound is an ester compound of the triprenylphenol compound obtained by using at least one compound selected from alcohols and carboxylic acids which have a carbon number of from 1 to 10.

9. The pharmaceutical composition for treatment or prevention of nephritis according to claim 1, wherein
the triprenylphenol compound is a water solvate.

10. A manufacturing method of a pharmaceutical composition for treatment or prevention of nephritis, the method comprising using a triprenylphenol compound as an active substance.

11. The manufacturing method of a pharmaceutical composition for treatment or prevention of nephritis according to claim 10, wherein:
the triprenylphenol compound is at least one selected from the group consisting of compounds represented by the following general formula (1), general formula (2) or formula (3). 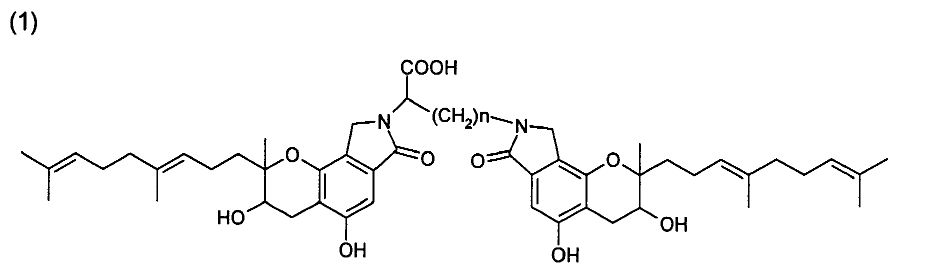 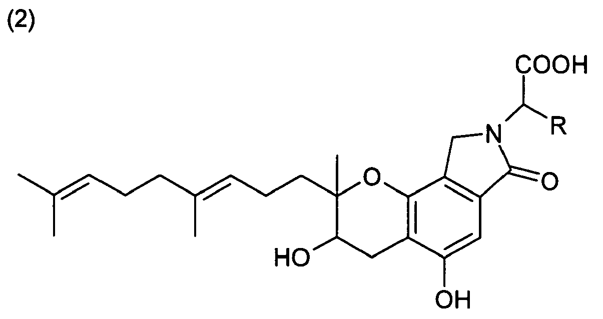 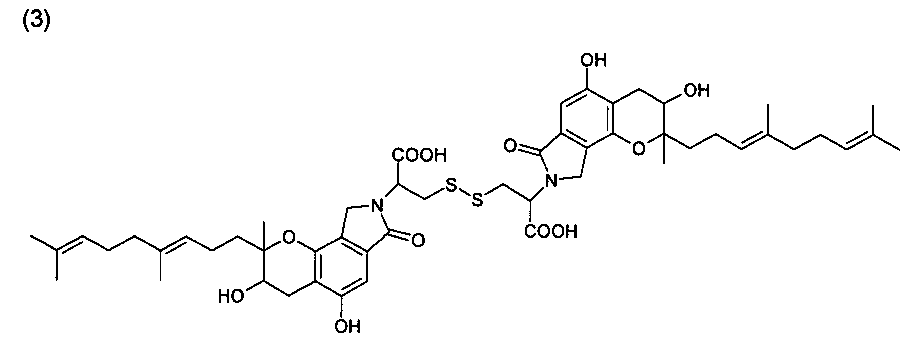 (In the above formule, n is an integer of from 1 to 1.0 and R is one of following.)

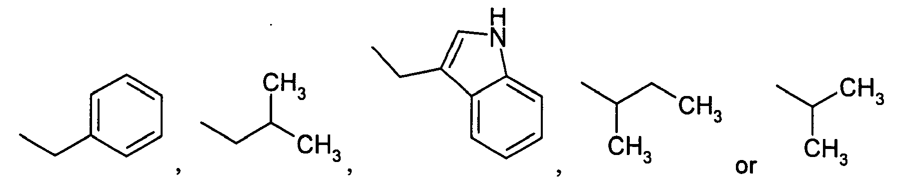

12. The manufacturing method according to claim 10; wherein
the triprenylphenol compound is at least one of the following compounds.

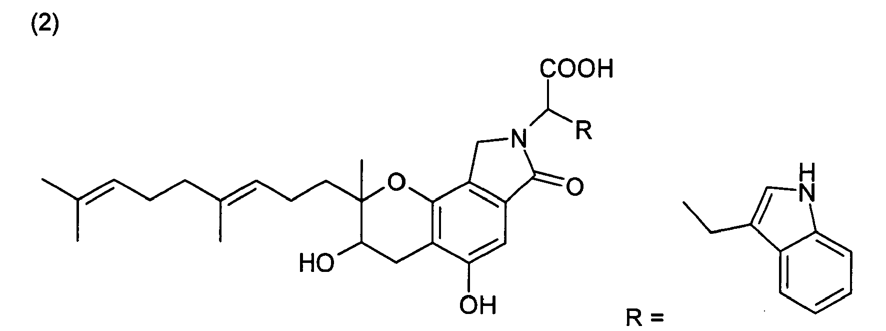

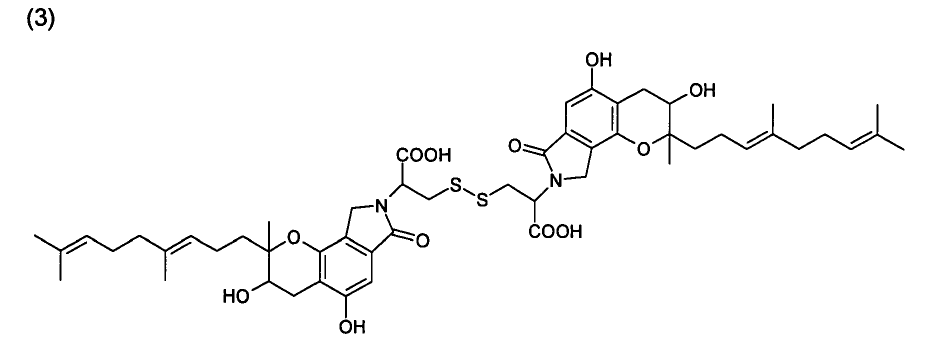

13. The manufacturing method according to claim 10; wherein
the triprenylphenol compound is the following compound.

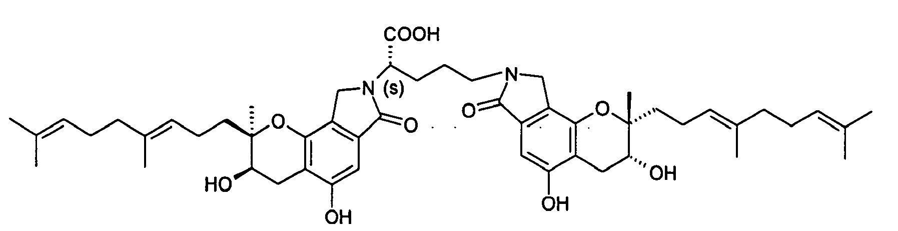

14. The manufacturing method according to any one of claims 10 to 12, further comprising:
incubating a filamentous fungus in a liquid medium, and
extracting said triprenylphenol compound from a culture.

15. The manufacturing method according to claim 13, wherein
the filamentous fungus is *Stachybotrys microspora,* IFO03001

16. The manufacturing method according to claim 13, further comprising:
incubating the filamentous fungus in a medium to which has been added at least one selected from amino acids, amino alcohols, and amines.

17. The manufacturing method according to claim 15, further comprising:
adding the at least one selected from amino acids, amino alcohols, and amines within three days from immediately after commencement of incubation.

18. The manufacturing method according to claim 15 or claim 16, wherein
an addition amount of the at least one selected from the amino acids and amino alcohols added to a medium is in the range of 0.5 mg/mL to 2 mg/mL.

19. The manufacturing method according to claim 15, wherein
the at least one selected from the amino acids and amino alcohols is at least one compound selected from the group consisting of L-ornitine, and compounds represented by the following general formula (4) and general formula (5).

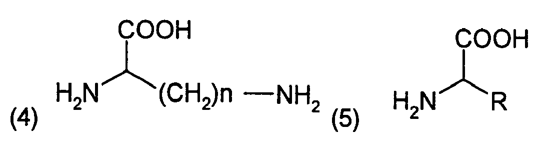

20. A manufacturing method of a pharmaceutical composition for treatment or prevention of nephritis comprising:
incubating *Stachybotrys microspora,* IFO030018 in a liquid medium;
adding L-ornitine to the medium within three days from immediately after commencement of incubation and incubating in the medium after addition,
extracting the triprenylphenol compound represented by the following formula from a culture after incubation, and
using the triprenylphenol compound extracted as an active substance of the pharmaceutical composition.
